# EUROPÄISCHE PATENTSCHRIFT

(11) **EP 1 579 811 B1**
(45) Veröffentlichungstag und Bekanntmachung des Hinweises auf die Patenterteilung: **07.01.2009**
(21) Anmeldenummer: 05006646.3
(22) Anmeldetag: 24.03.2005
(51) Int. Cl.: A61B 19/02, B65D 43/02, B65D 51/24, B65F 1/16

(54) **Abfallbehälter**
Refuse receptacle
Poubelle

(30) Priorität: 24.03.2004 DE 102004014826
(43) Veröffentlichungstag der Anmeldung: 28.09.2005
(73) Patentinhaber: Udo Heisig GmbH, 85640 Putzbrunn (DE)
(72) Erfinder: Heisig, Udo, 85640 Putzbrunn (DE)
(74) Vertreter: Feldkamp, Rainer

(56) Entgegenhaltungen:
- EP-A- 0 403 704
- DE-C1- 10 236 378
- US-A- 4 032 037
- US-A- 5 829 588
- US-A- 5 947 285

## Beschreibung

Die Erfindung bezieht sich auf einen Abfallbehälter der im Oberbegriff des Anspruchs 1 genannten Art.

Derartige Abfallbehälter werden häufig in medizinischen Einrichtungen verwendet, wobei der Behälterdeckel Abstreiföffnungen beispielsweise für Kanülen und dergleichen aufweist. Wenn derartige Abfallbehälter zu stark befüllt werden, so ergibt sich ein erhöhtes Risiko dadurch, dass Kanülen und andere spitze Gegenstände mit zu hoher Gewalt in den Abfallbehälter eingedrückt werden, so dass diese Gegenstände die Außen- oder Bodenwand des Abfallbehälters durchdringen können und somit ein erhebliches Sicherheitsrisiko darstellen.

Um eine übermäßige Befüllung zu vermeiden und dem Anwender einen eindeutigen Hinweis auf den Füllzustand zu geben, ist es üblich, den Abfallbehälter durchsichtig auszubilden oder in dem Abfallbehälter Klarsichtfenster mit einer entsprechenden Markierung vorzusehen, was jedoch einen erheblichen Aufwand in der Behälterfertigung darstellt und einen in vielen Fällen unerwünschten Einblick in das Behälterinnere gestattet.

Wenn der Behälterkörper opak eingefärbt ist, ist es jedoch schwierig, den Füllstand zu kontrollieren.

Bei Abfallbehältern ist es auch üblich, einen nach innen abklappbaren Deckel zu verwenden. Solche Behälter sind beispielsweise aus der US 4 032 037 und US 5 947 285 bekannt. Bei diesen Behältern ist ein Öffnen des Deckels nicht mehr möglich, sobald der Behälter voll ist. Eine Kontrolle des Füllstandes ist jedoch erst zu diesem Zeitpunkt möglich.

Der Erfindung liegt die Aufgabe zugrunde, einen Abfallbehälter der eingangs genannten Art zu schaffen, der eine verbesserte Kontrolle des Füllstandes ermöglicht.

Diese Aufgabe wird durch die im Patentanspruch 1 angegebenen Merkmale gelöst.

Vorteilhafte Ausgestaltungen und Weiterbildungen der Erfindung ergeben sich aus den Unteransprüchen.

Durch die erfindungsgemäße Ausgestaltung des Abfallbehälters ist es durch Betrachten des sich unterhalb der Einfüllöffnung des Abfallbehälters befindlichen Füllstands-Anzeigeelementes sehr leicht möglich, den Füllstand zu kontrollieren, da spätestens dann, wenn der Füllstand die für eine bestimmte Behältergröße vorgesehene Abstufung auf dem Füllstands-Anzeigeelement erreicht, der Füllvorgang beendet werden muss.

Das Füllstands-Anzeigeelement kann bei der Fertigung des Behälterdeckels einstückig mit diesem ausgebildet werden und vorzugsweise über ein Schamierelement mit einem Rand der Einfüllöffnung des Behälterdeckels verbunden sein. Auf diese Weise bleiben die Behälterdeckel stapelbar, und andererseits kann das Füllstands-Anzeigeelement vor dem Aufsetzen des Behälterdeckels auf den Behälterkörper in etwa rechtwinklig zu der Ebene des Behälterdeckels abgeklappt und in dieser Stellung über gegebenenfalls vorgesehene Rasteinrichtungen verriegelt werden.

Die Erfindung wird im Folgenden anhand von in der Zeichnung dargestellten Ausführungsbeispielen noch näher erläutert.

In der Zeichnung zeigen:
Figur 1 ein Beispiel eines Abfallbehälters, auf den die Erfindung anwendbar ist,
Figur 2 eine Draufsicht auf den Behälterdeckel des Abfallbehälters nach Figur 1, der das Füllstands-Anzeigeelement vor dem Abklappen erkennen lässt,
Figur 3 eine vergrößerte Ansicht von oben auf einen Teilbereich des Behälterdeckels , die das Füllstands-Anzeigeelement vor dem Abklappen in eine zum Behälterdeckel im Wesentlichen senkrechte Stellung zeigt,
Figur 4 eine Seitenansicht der Figur 3
Figur 5 eine Unteransicht des in Figur 3 gezeigten Teilbereichs,
Figur 6 eine perspektivische Ansicht, die die Rasteinrichtungen erkennen läßt.

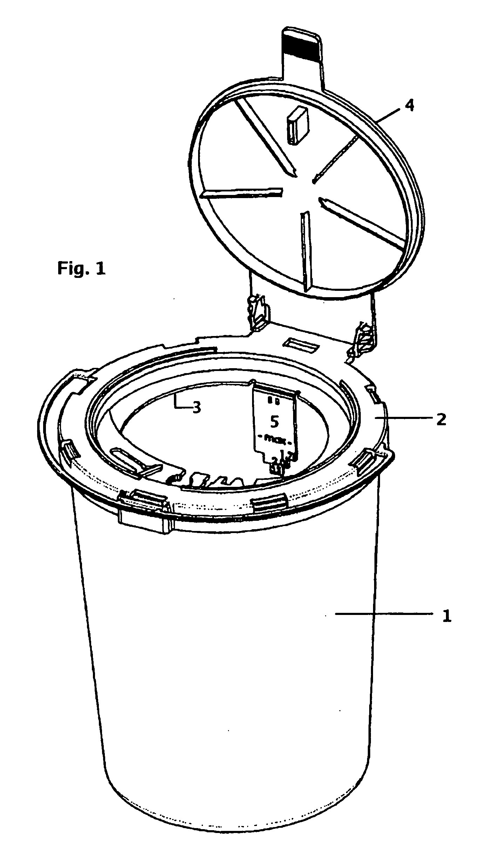

In Figur 1 ist ein Ausführungsbeispiel eines Abfallbehälters dargestellt, auf den die vorliegende Erfindung anwendbar ist. Dieser Abfallbehälter weist einen Behälterkörper 1 und einen auf diesen aufrastbaren Behälterdeckel 2 auf, der eine Einfüllöffnung 3 aufweist, die getrennt durch einen im geöffneten Zustand gezeigten Verschlussdeckel 4 verschließbar ist.

An einem Randbereich der Einfüllöffnung 3 ist ein Füllstands-Anzeigeelement 5 angeordnet, das in Figur 1 in der Gebrauchsstellung gezeigt ist

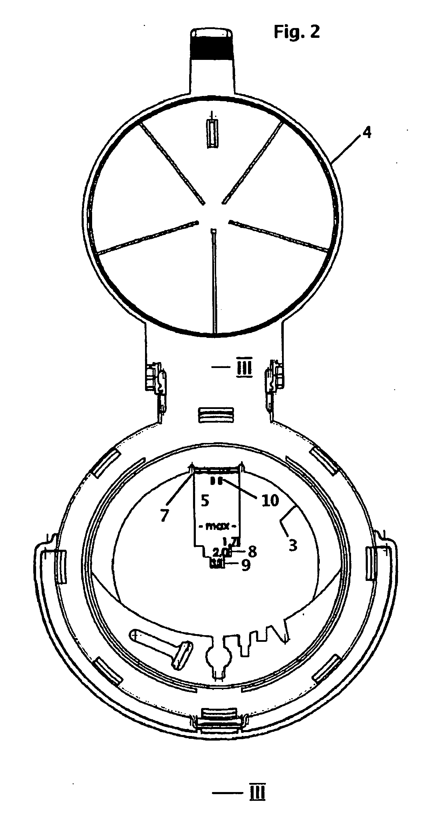

Figur 2 zeigt eine Draufsicht auf den Behälterdeckel 2, in dessen Einfüllöffnung 3 das Füllstands-Anzeigeelement 5 angeordnet ist, das an der Randkante der Einfüllöffnung 3 über ein Filmschamier 7 abklappbar befestigt ist. Das Füllstands-Anzeigeelement weist an seinem freien Ende abgestufte Gradierungen 8, 9 auf, die bei im Wesentlichen senkrecht zur Ebene des Behälterdeckels und in den Behälterkörper 1 abgeklappten Füllstands-Anzeigeelement 5 eine Beobachtung der Füllstandshöhe ermöglichen.

Bei der dargestellten Ausführungsform weist das Füllstands-Anzeigeelement 5 zwei Abstufungen auf, die beispielsweise den maximalen Füllstand für jeweils unterschiedliche Behältergrößen anzeigen, mit denen der Behälterdeckel verwendet werden kann. Selbstverständlich kann eine beliebige Anzahl von Füllstandsmarken vorgesehen werden.

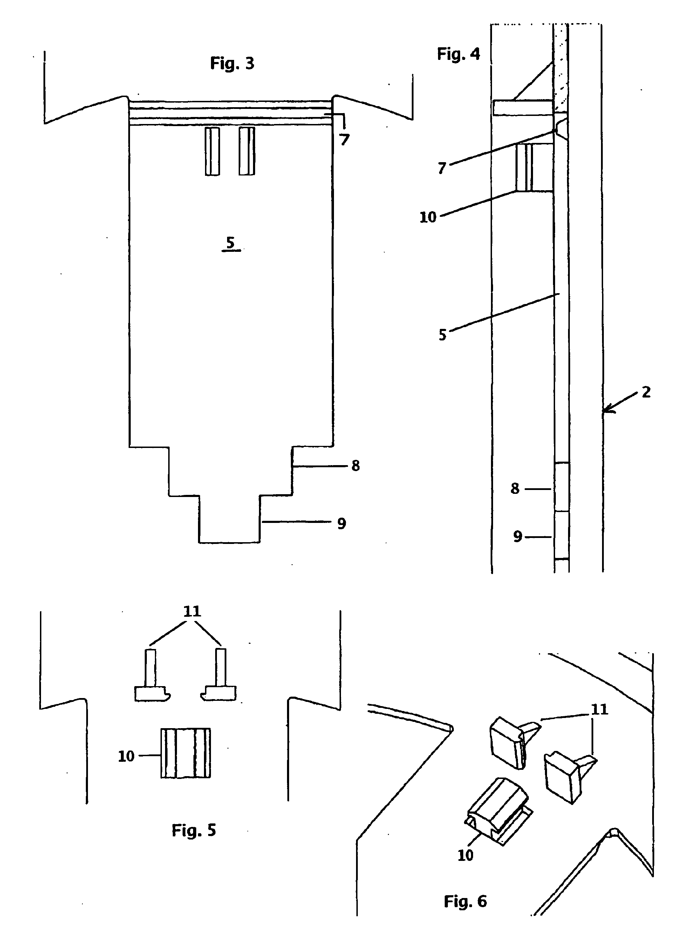

Das Füllstands-Anzeigeelement kann durch Rastelemente 10, 11 an dem Füllstands-Anzeigeelement 5 und dem Behälterdeckel 2 in der im Wesentlichen zum Behälterdeckel senkrechten Ebene verriegelt werden, wobei dieses Abklappen und Verriegeln vorzugsweise vor dem Aufsetzen des Behälterdeckels auf den Behälterkörper erfolgt, wie dies insbesondere aus den Figuren 3 und 6 zu erkennen ist.

Zu diesem Zweck ist an dem Füllstands-Anzeigeelement 5 auf dessen Unterseite benachbart zu einer Seite des Filmscharniers 7 eine elastische Rastnase 10 angebracht, die mit zwei benachbarten Rastvorsprüngen 11 zusammenwirken kann, die auf der anderen Seite des Filmschamiers 7 auf der Unterseite des Behälterdeckels 2 vorgesehen sind. Wenn das Füllstands-Anzeigeelement 5 nach unten hin abgeklappt wird, greift die Rastnase 10 zwischen die Rastvorsprünge 11, und das Füllstands-Anzeigelement 5 wird in der nach unten hin abgeklappten Gebrauchsstellung verriegelt.

## Patentansprüche

1. Abfallbehälter, insbesondere für medizinische Abfälle, mit einem Behälterkörper (1) und einem auf diesen aufrastbaren Behälterdeckel (2), der eine Einfüllöffnung (3) aufweist, die getrennt durch einen Verschlussdeckel (4) verschließbar ist, wobei der Behälterdeckel (2) im Bereich der Einfüllöffnung (3) ein Füllstands-Anzeigeelement (5) aufweist, das bei auf den Behälterkörper (1) aufgesetzten Behälterdeckel (2) im Wesentlichen senkrecht zu diesem abklappbar ist und sich in das Behälterinnere erstreckt, **dadurch gekennzeichnet, dass** das Füllstands-Anzeigeelement (5) im Gebrauch eine sich nach unten hin verjüngende abgestufte Form aufweist, wobei die Abstufungen (8, 9) Gradierungen zur Anzeige der Füllstandshöhe bilden.

2. Abfallbehälter nach Anspruch 1, **dadurch gekennzeichnet, dass** das Füllstands-Anzeigeelement (5) über ein Scharnierelement (7) mit einer Randkante der Einfüllöffnung (3) verbunden ist.

3. Abfallbehälter nach einem der vorhergehenden Ansprüche, **dadurch gekennzeichnet, dass** an dem Behälterdeckel (2) und dem Füllstands-Anzeigeelement (5) zusammenwirkende Rasteinrichtungen (10, 11) vorgesehen sind, die das Füllstands-Anzeigeelement (5) in der im Wesentlichen senkrecht zum Behälterdeckel (2) verlaufenden Stellung im Inneren des Behälterkörpers (1) halten.

## Claims

1. A waste container, in particular for medical waste, having a container body (1) and a container lid (2) that snaps onto said container body, which lid comprises a filler opening (3) that can be closed separately by a cover (4), in the region of the filler opening (3) the container lid (2) comprising a level display element (5), which can swing substantially perpendicular to the container lid (2) when said lid is placed on the container body (1), and which extends into the interior of the container,
**characterised in that** during use the level display element (5) comprises a downwardly tapering stepped shape, with the stepped portions (8, 9) forming graduations to display the filling level.

2. A waste container according to Claim 1,
**characterised in that** the level display element (5) is connected to an edge of the filler opening (3) by a hinge element (7).

3. A waste container according to one of the preceding Claims,
**characterised in that** catch mechanisms (10, 11) interacting at the container lid (2) and the level display element (5) are provided, which hold the level display element (5) in the position running substantially perpendicularly to the container lid (2) in the interior of the container body (1).

## Revendications

1. Poubelle, en particulier pour des déchets médicaux, avec un corps de récipient (1) et avec un couvercle de récipient (2) qui peut être enclenché sur ce corps et qui présente une ouverture de remplissage (3) qui peut être fermée séparément par un couvercle de fermeture (4), sachant que le couvercle de récipient (2) présente dans la région de l'ouverture de remplissage (3) un élément (5) indicateur de niveau de remplissage qui, lorsque le couvercle de récipient (2) est en place sur le corps de récipient (1), peut être rabattu sensiblement perpendiculairement à ce couvercle, et s'étend à l'intérieur du récipient, **caractérisée en ce que** l'élément (5) indicateur de niveau de remplissage présente en utilisation une forme échelonnée se rétrécissant vers le bas, sachant que les échelonnements (8, 9) forment des graduations pour indiquer le niveau de remplissage.

2. Poubelle selon la revendication 1, **caractérisée en ce que** l'élément (5) indicateur de niveau de remplissage est relié par l'intermédiaire d'un élément formant charnière (7) à une arête de bord de l'ouverture de remplissage (3).

3. Poubelle selon l'une des revendications précédentes, **caractérisée en ce que** des moyens d'enclenchement (10, 11) coopérant entre eux sont prévus sur le couvercle de récipient (2) et sur l'élément (5) indicateur de niveau de remplissage, lesquels maintiennent l'élément (5) indicateur de niveau de remplissage à l'intérieur du corps de récipient (1) dans la position s'étendant sensiblement perpendiculairement au couvercle de récipient (2).
